# EUROPEAN PATENT SPECIFICATION

(11) **EP 0 597 997 B1**
(45) Date of publication and mention of the grant of the patent: **20.05.1998**
(21) Application number: 92917175.9
(22) Date of filing: 06.08.1992
(51) Int. Cl.: C07K 7/06, C07K 7/08, C07K 14/00, C07K 7/16, C07K 1/04, A61K 38/00

(54) **LANTHIONINE BRIDGED PEPTIDES**
PEPTIDE MIT EINER LANTHIONIN-BRÜCKE
PEPTIDES RELIES PAR DES PONTS LANTHIONINE

(30) Priority: 09.08.1991 US 742908
(43) Date of publication of application: 25.05.1994
(73) Proprietor: KOLBECK, Winfried, D-81247 München (DE)
(72) Inventor: KOLBECK, Winfried, D-8000 München 60 (DE); GOODMAN, Murray, La Jolla, CA 92037 (US); OSAPAY, George, La Jolla, CA 92122 (US)
(74) Representative: Keller, Günter, Dr.
(86) International application number: EP9201789
(87) International publication number: WO9303056

(56) References cited:
- EP-A- 0 113 029
- FR-A- 2 320 109
- PEPTIDES. CHEMISTRY, STRUCTURE AND BIOLOGY. PROCEEDINGS OF THE XI AMERICAN PEPTIDE SYMPOSIUM, JULY 9-14, LA JOLLA, CA, USA. 1990, ESCOM, LEIDEN pages 443 - 445 M F BEAN ET AL. 'identification of a thioether by-product in the synthesis of a acyclic disulfide peptide by tandem mass spectrometry' cited in the application
- TETRAHEDRON LETTERS. vol. 25, no. 20, 1984, OXFORD GB pages 2067 - 2068 M LEBL ET AL 'synthesis of cyclic peptides by solid phase methodology'
- COLLECTION OF CZECHOSLOVAK CHEMICAL COMMUNICATIONS. vol. 39, no. 10, October 1974, PRAGUE CS pages 2835 - 2856 K JOST ET AL. 'synthesis and some biological activities of analogues of deamino-vasopressin with the disulphide bridge altered to a thioether bridge'
- PEPTIDES. CHEMISTRY, STRUCTURE AND BIOLOGY. PROCEEDINGS OF THE XI AMERICAN PEPTIDE SYMPOSIUM, JULY 9-14, LA JOLLA, CALIFORNIA, USA 1990, ESCOM, LEIDEN pages 865 - 869 G JUNG 'peptides with sulfide bridges and dehydroamino acids: their prepropeptides and possibilities for bioengineering' cited in the application
- JOURNAL OF THE AMERICAN CHEMICAL SOCIETY. vol. 114, no. 14, 1 July 1992, GASTON, PA US pages 5634 - 5642 D E PALMER ET AL. 'effects of dehydroalanine on peptide conformation'

## Description

It is a basic goal in peptide chemistry to design molecules for medical or industrial application. Design means that naturally occurring peptides which have a biological activity are modified in order to obtain molecules which have advantages over the naturally occurring peptides in different respects. There are several groups of peptides which act as hormones, as neurotoxins or as plant regulating agents. These peptides are usually small, flexible molecules which may optionally have a disulfide bridge.

It is an object of the present invention to provide peptides which comprise a monosulfide bridge. This thioether bond is also designated as a lanthionine bridge and corresponds with the cystine bridge with the exception that the disulfide bridge is replaced by a monosulfide linkage. Two amino acid residues having the general formula 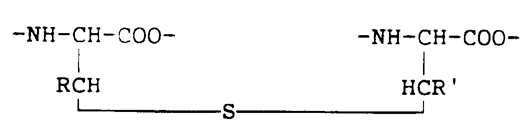 are designated to be joined into a lanthionine bridge wherein the linkage of the two amino acids has the meaning -RCH-S-HCR'-, wherein R and R' respectively represent -H, a lower (C₁-C₁₀) alkyl or aralkyl group. In a preferred embodiment R and R' are H. The amino acid termini of the lanthionine structure are designated as Ala_{L} if R and R' are H and Thr_{L} when R or R' are CH₃. Other β-substituted lanthionine components are designated as substituted Ala_{L} derivatives, e.g. βethylAla_{L}.

Thioether bonds of the lanthionine type are known from some fungal toxins and antibiotics, for example from the lantibiotics, as nisin, epidermin, dunamycin or mersacidin. Naturally occurring compounds having the monosulfide bridge always have more than two monosulfide bridges in the molecule.

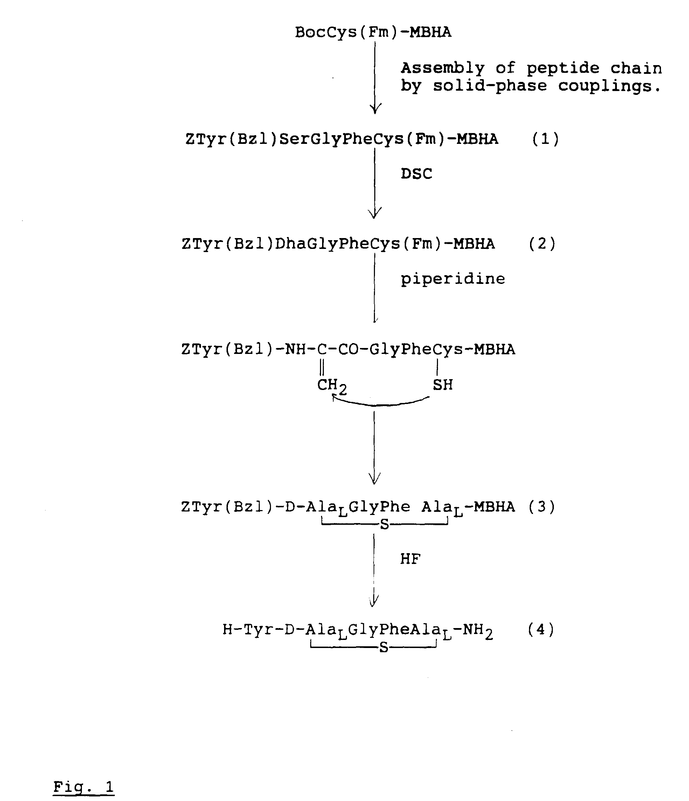

M.F. Bean et al. have reported in their article "Identification of a Thioether By-product in the Synthesis of a Cyclic Disulfide Peptide by Tandem Mass Spectrometry" as published in the Proceedings of the 11th American Peptide Symposium, ESCOM, (Leiden 1990, p. 443) on a somatostatin analog wherein the internal disulfide bond has been converted to a thioether link. The somatostatin analog with the putative amino acid sequence Phe-Ala-Phe-Trp-Lys-Thr-Ala-Thr(ol), wherein the two Ala_{L} residues are linked via the thioether bridge and wherein on page 444, figure 1 "Thr(ol)" is disclosed as -NHCH(CH₂OH)CHOHCH₃, has been described as the by-product which was obtained by the Boc-TFA-preparation of sandostatin analogs. The originally occurring somatostatin derivative has a disulfide bridge.

It is an object of the present invention to provide analogs of peptide compounds having at least one monosulfide bridge in the molecule and exhibiting an improved biological activity. The analogs of peptide compounds according to the invention comprise analogs of compounds, such as: ACTH-analogs, angiotensines, magainine, bombesine, bradykinine, fragments of fibronectine, CCK, fragments of hirudine, LHRH-analogs, neuropeptides, neurokinines, neurotensines, substance P, virus related peptides, such as peptides of HIV, thymosine, fragments of thymopoeitin, fragments of oncogenes, atrial natriuric peptides, epidermal growth factors, transforming growth factor and fragments thereof, conotoxines and related neurotoxines, mast cell degranulating peptides (MCD), urotensine II, HIV gp41 antigenic peptide 1 or peptide 4 or tyrocidin A.

In a preferred embodiment of the present invention the peptide has not more than two monosulfide bridges and in an especially preferred embodiment the peptide has only one monosulfide bridge.

The compounds of the present invention have a higher biological activity than the corresponding naturally occurring peptides.

According to the present invention lanthionine-bridged peptides are disclosed having the general formula 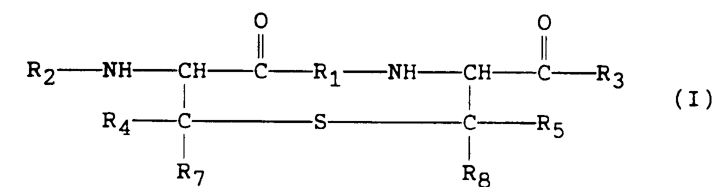 wherein R₁ is a short sequence of 2 to 10 amino acids selected from the group of the naturally occurring amino acids and the D-enantiomers thereof and

R₂ and R₃ respectively represent naturally occurring amino acids as L- or D-enantiomers or a short sequence of up to 25, preferably 3 amino acids, wherein the N-terminal -NH₂ group of the R₂ residue may be replaced by -OH, -H or -NHCOR₆ wherein R₆ is an alkyl- or aralkylresidue or the C-terminal -COOH of the R₃ amino acid residue may be replaced by -CONH₂ or -CH₂OH or R₂ may represent -H, acyl or aracyl each of them having 1-18 carbon atoms and R₃ may be -OH or -NH₂ and 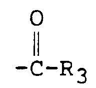 may be replaced by CH₂OH with the proviso that R₁ is not Phe-Trp-Lys-Thr, when R₂ is Phe and R₃ is Thr(ol), whereby R₁, R₂ or R₃ can also comprise peptidomimetics such as retro-inverso-, carba-, aza-, thiopeptides or peptide rings and wherein R₄, R₅, R₇ and R₈ are hydrogen or an alkyl having 1 to 10 carbon atoms.

In a preferred embodiment of the present invention R₄, R₅, R₇ and R₈ represent hydrogen or a methyl group, wherein each of R₄, R₅, R₇ and R₈ may be a methyl group.

The amino acids can be selected from the group consisting of the naturally occurring amino acids including the L-enantiomers and the D-enantiomers. The group of the naturally occurring amino acids comprises alanine, arginine, asparagine, aspartic acid, cysteine, cystine, glutamic acid, glutamine, glycine, histidine, hydroxyproline, isoleucine, leucine, lysine, methionine, phenylalanine, proline, serine, threonine, tryptophan, tyrosine, valine, β-alanine, γ-aminobutyric acid, betaine, carnitine, citrulline, creatine, ornithine, saccharopine, 3,4-dihydroxyphenylalanine, 5-hydroxytryptophan, thyroxine, homocysteine, S-methylmethionine, penicillamine, pipecolic acid and nalidixic acid.

The radicals R₁, R₂ or R₃ can also comprise peptidomimetics such as retro-inverso-, carba-, aza-, thiopeptides or peptide rings.

It is understood that a regular peptide has the following structure: 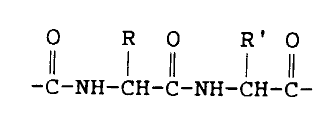 whereas the retro-inverso-structure has the following formula:

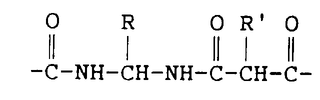

In a preferred embodiment of the present invention the peptides according to the invention of formula (I) have a short sequence of 2 to 7 and most preferably 2 to 4 amino acids representing residue R₁.

Preferably the amino acids of the residues R₂ and R₃ are selected from the group of amino acids comprising D-Phe, D-β-Nal, Tyr, TrpNH₂, ThrNH₂, Thr(ol). Alternatively the substituents R₂ can be H, acyl or aracyl with 1 to 18, preferably 2-12 carbon atoms and R₃ can have the meaning of -OH or -NH₂ and 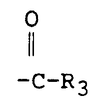 can be replaced by CH₂OH. Moreover R₂ and R₃ respectively can be a short amino acid sequence of Pro-Arg-Gly or Pro-Leu-Gly.

In preferred embodiments of the present invention R₁ is represented by a short amino acid sequence selected from the group consisting of Gly-Phe; Phe-D-Trp-Lys-Thr; Phe-D-Trp-Lys-Val; Tyr-Phe-Gln-Asn, Tyr-Ile-Gln-Asn, Tyr-D-Trp-Lys-Val, Gly-Asn-Leu-Ser-Thr, Ser-Asn-Leu-Ser-Thr or Glu-Lys-Asp-Met-Leu-Ser-Ser.

Among the especially preferred peptides of the present invention are: lanthionine-enkephalins having the formula 
H-Tyr-D/L-Ala_{L}-Gly-Phe-D/L-Ala_{L}-R₃
(II)
S
 wherein R₃ is OH or NH₂,

lanthionine-somatostatins having the general formula 
H-Xxx-Ala_L-Phe-D-Trp-Lys-Yyy-Ala_L-Zzz
S
(III)
 wherein Xxx = D-Phe, D-β-Nal; Yyy = Thr, Val; Zzz = TrpNH₂, ThrNH₂ or Thr(ol) with the proviso that Xxx is not Phe when Zzz is Thr(ol) and Yyy is Thr.

Lanthionine-vasopressin having the formula 
H-Ala_L-Tyr-Aaa-Gln-Asn-Ala_L-Pro-Bbb-Gly-NH_2          (IV)
S
 wherein Aaa = Phe and Bbb = Arg, or
lanthionine-oxytocin 
H-Ala_{L}-Tyr-Aaa-Gln-Asn-Ala_{L}-Pro-Bbb-Gly-NH₂ (V)
S
 wherein Aaa = Ile and Bbb = Leu.

Further preferred peptides of the present invention have the formula (VI) 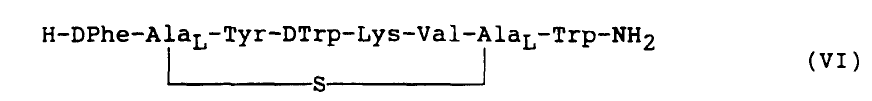
H-DPhe-AlaL-Tyr-DTrp-Lys-Val-AlaL-Trp-NH2          (VI)
 S
 or the formula (VII)

H-DßNal-Ala_{L}-Tyr-DTrp-Lys-Val-Ala_{L}-Thr-NH₂
(VII)
S

Further preferred peptides have the general formula (VIII) 
R2-AlaL-Ggg-Asn-Leu-Ser-Thr-AlaL-R3
S
(VIII)
 wherein R₂ is H, acyl or aracyl, R₃ is the fragment 8-32 of human, salmon or eel-calcitonin and Ggg is Gly or Ser.

In another preferred embodiment of the present invention the peptide has the amino acid sequence of endothelin (Schematic Structure A see below) wherein one or two of the naturally occurring disulfide bridges are replaced by a thioether bond. Therefore the compounds can be shown as described by the schematic structures B, C and D.

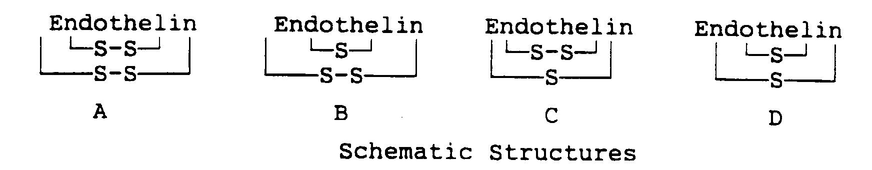

The preferred peptides of the present invention will have sequentially overlapping thioether bonds as shown above, if the peptide has two thioether linkages. This means that in the amino acid sequence one lanthionine-bridge is located between two Ala_{L} residues forming the second lanthionine-bridge.

The peptides of the present invention can be used as pharmaceutically active compounds. They can therefore be used in pharmaceutical compositions comprising at least one of the peptides of the present invention.

Depending on the nature of the biologically active peptide they can be used in injection solutions, capsules, tablets, ointments, creams, sprays and suppositories.

A representative example of the peptides of the present invention can be produced according to the following procedure described for the enkephalin

S
H-Tyr-D-Ala_{L}GlyPheAla_{L}NH₂

The linear peptide chain was assembled on a methylbenzhydrylamine resin using *tert*-butoxycarbonyl chemistry with the symmetrical anhydride peptide coupling method. A serine residue was preferably incorporated at position 2 and later on converted to dehydroalanine using disuccinimido carbonate. The S-protecting group (fluorenyl methyl) was selectively removed with piperidine.

A slightly basic milieu, preferably 5% piperidine/dimethylformamide, promoted the Michael addition of the SH-group to the double bond. The amino acid analysis showed 48% conversion of serine. A greater excess of the reagent disuccinimido carbonate would have resulted in an increased yield for these two steps. "Low-high HF cleavage" was used to cleave the peptide from the resin and to remove the protecting groups. Purification of the resultant crude product was achieved by preparative RP-HPLC using a gradient acetonitrile-water elution. The material obtained was further purified and desalted by gel filtration on a Sephadex G-15 column (20% acetic acid/water).

The product was identified by amino acid analysis and mass spectrometry. Although the Michael addition is not stereoselective, this reaction resulted in only the (2D, 5L) diastereomer. The other diastereomer expected, (2L, 5L) could not be detected in the reaction mixture. Steric hindrance from the solid support near the SH group may be responsible for the stereoselectivity of the addition reaction. The solid phase synthetic approach allows a rapid assembly of lanthionine-bridged cyclopeptides. Figure 1 shows schematically the process according to the invention. The following examples illustrate the present invention.

### Abbreviations used in peptide synthesis section

Standard abbreviations for amino acids and protecting groups are followed according to the IUPAC-IUB Joint Commission on Biochemical Nomenclature: *J. Biol. Chem. 1971*, 247, 977. Abbreviations used: Acm, amidocarboxymethyl; Boc, *ter*-butoxycarbonyl; Bzl, benzyl; DCC, *N,N*'-dicyclohexylcarbodiimide; DCM, dichloromethane; Dha, dehydroalanine; DMF, dimethylformamide; DIEA, *N,N*-diisopropylethylamine; DSC, disuccinimido carbonate; EtOAc, ethyl acetate; Fm, fluorenylmethyl; Fmoc, fluorenylmethyloxycarbonyl; HOBt 1-hydroxybenztriazole; Ala_{L}, lanthionine; MBHA, methylbenzhydrylamine resin; Pac, phenylacyl; TFA, trifluoroacetic acid; Tmse, trimethylsilylethyl; Z, benzyloxycarbonyl; NCA, N-carboxyanhydrid; Trt, trityl.

### Experimental procedures

All amino acids were of the **L**-configuration except as indicated. Protected amino acids were purchased from Bachem, Inc. ACS grade solvents (DCM, DMF, acetonitrile) were purchased from Fisher Scientific and purged with nitrogen, then stored over molecular sieves from Sigma. DIEA (Aldrich) was dried over KOH and distilled from ninhydrin. MBHA resin.HCl (Calbiochem) was swollen in DCM and washed with 5% DIEA/DCM followed by DCM before use. TFA, piperidine, DSC (Aldrich) and DCC (Fluka) were used without further purification. Silica gel for flash chromatography was purchased from Baker.

Peptides were analyzed on precoated silica gel 60F-254 plates (Merck) using (A) chloroform:methanol:acetic acid, 65:35:1; (B) butanol:acetic acid:water, 4:1:5 - upper phase. Compounds were visualized by UV, ninhydrin, chlorine/o-tolidine and KMnO₄ solution. RP-HPLC analyses were performed on a Waters (Model 510 and Waters 484 detector) instrument with a C-18 analytical column.

There is, however, another synthetic method for the production of peptides. It is often desirable to have diastereomeric peptide analogs. The use of diastereomeric mixture of lanthionine units can provide appropriate diastereomeric analogs, separable by chromatography (HPLC). By such routes, two (or four) analogs can be prepared by a single synthetic process. The simultaneous application of the benzyloxycarbonyl, t-butyloxycarbonyl and phenacyl (or methyl, trimethylsilylethyl, etc.) groups defines the synthetic strategy of this invention to prepare 
S
Z-AlaL(Boc-AlaL-OPac)-OH.
 A new application of the PCOR method (Peptide Cyclization on an Oxime Resin) can provide the new cyclic segment containing a defined lanthionine bridge (Scheme 2), where the chain can be elongated at both termini. The prdcess without a lanthionine bridge has been described in more detail by Osapay et al. in J. Am. Chem. Soc. 1990, 112, p. 6046-6051 and Tet. Lett. 1990, 31, p. 6121-6124. The final deprotection step followed by chromatographic purification yields the desired compounds.

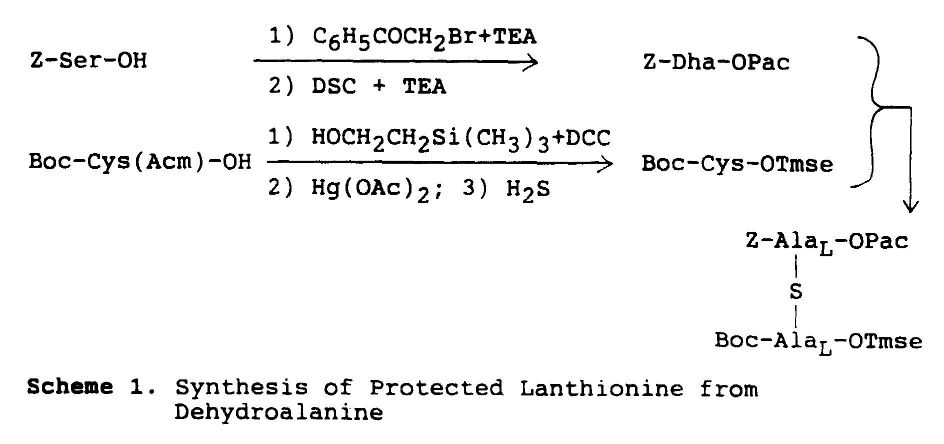

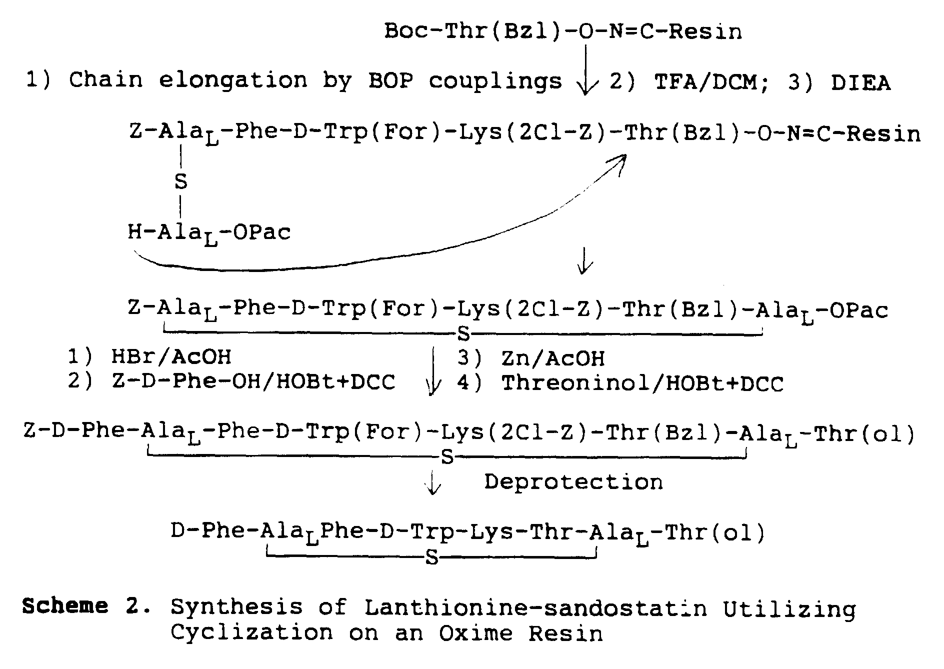

Another highly promising pathway involves the synthesis of two protected intermediates, followed by coupling of the two components in generating an optically pure lanthionine. This is proceeded by the synthesis of both the protected serine β-lactone (Arnold et al. J. Am. Chem. Soc. 1988, 110, p. 2237-2241) and the protected cysteine. The latter compound acts as a nucleophile in opening the lactone ring at the site of the methylene group (see **Scheme 3**).

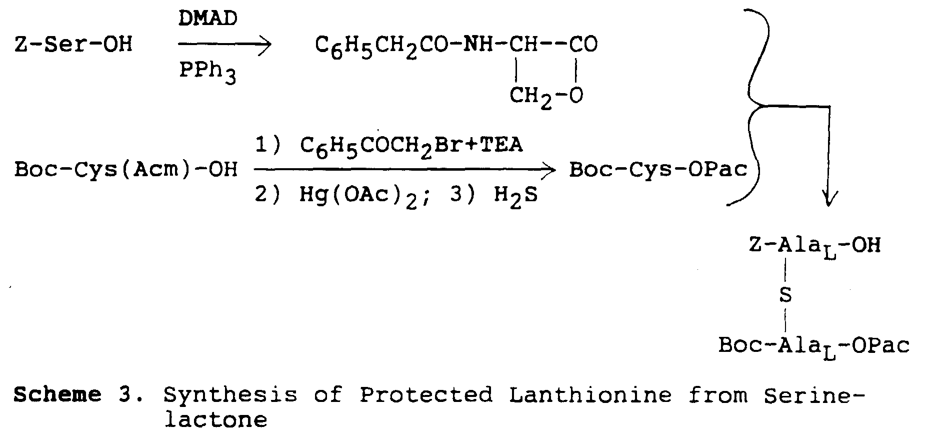

Furthermore another route for the synthesis of the protected lanthionine is disclosed where reactions proceed with retention of configuration. This lanthionine derivative is prepared through the ring opening of an aziridine derivative (Wakamiya et al., Bull. Chem. Soc. Jpn., 1982, 55, 3878-3881) by a nucleophile, namely cysteine or any appropriate SH-containing amino acid (**Scheme 4**).

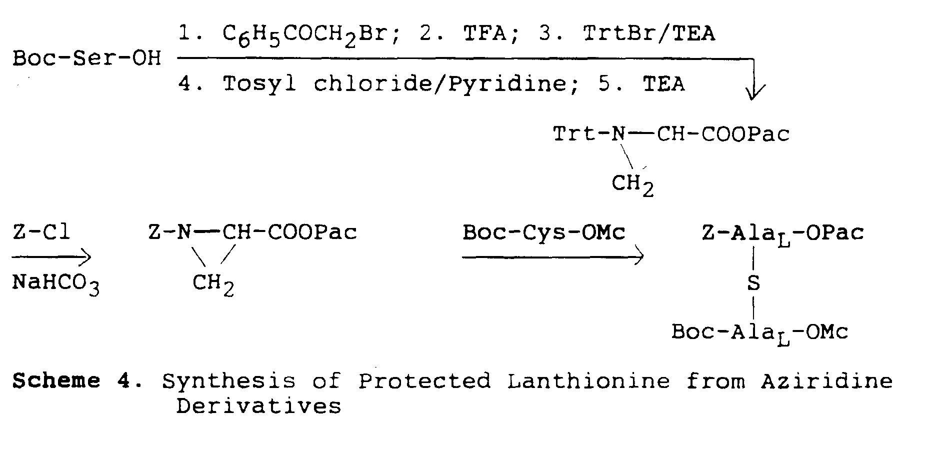

### Preparation of Lanthionine-opioids

As will be shown later, all of the lanthionine opioids synthesized are superactive both at the µ- and δ -receptor. To investigate structural or pharmacochemical aspects of this new class of opioids, analogs can be synthesized in order to carry out bioassays and conformational analyses of the resulting molecules. Various peptidic or peptidomimetic units can be incorporated into cyclic enkephalin and dermorphin-deltorphin structures including: 
S                              S
Tyr-[D-Ala_{L}-Gly-Phe-Ala_{L}]-X, Tyr-[D-Ala_{L}-Phe-Ala_{L}]-X and
 
S
Tyr-[D-AlaL-Phe-Asp-AlaL]-X (X = NH2 or OH).
 The incorporation of methyl group(s) at the β-carbon(s) and effects of chirality at the two main chain units of the lanthionine residue can also be included.

Thus the synthesis of β-methyl lanthionines and β,β-dimethyl lanthionine results in modifications, which are expected to lead to substantial differences in bioactivity profiles for closely related target molecules. Thus, critical information about the "bioactive conformations" of the analogs can be obtained. In addition, specific residues such as the Gly in the 
Tyr-[D-AlaL-Gly-Phe-AlaL]-X
S
 and the Asp of 
S
Tyr-[D-AlaL-Phe-Asp-AlaL]-X
 can be modified with natural and unnatural amino acids. This family of opioids are most promising for obtaining novel and clinically useful opioid drugs.

### Lanthionine-somatostatins

New lanthionine-somatostatin derivatives can be synthesized. First, the cyclic segment with a monosulfide bridge of somatostatin or "key-hexapeptide" (**Scheme 2**) or other analogs of somatostatin according to the definition of R₁ on a Kaiser-oxime resin has to be prepared. It will be elongated at both termini (D-Phe at the N-terminus and threoninol at the C-terminus) to obtain for example the lanthionine analog of Sandostatin. The same synthetic strategy can be used for the preparation of the lanthionine analog of the native somatostatin tetradecapeptide. Potency and receptor selectivity of both target molecules are promising.

D-Phe-Ala_{L}-Phe-D-Trp-Lys-Thr-Ala_{L}-Thr(ol)
S
Lanthionine-sandostatin

AlaGlyAla_{L}LysAsnPhePheTrpLysThrPheThrSerAla_{L}
S
Lanthionine-somatostatin

### Lanthionine-calcitonins

It is possible to incorporate the lanthionine as the replacement of the cysteine-cysteine disulfide bridge in the N-terminal loop. The loop can be prepared via the PCOR method (**Scheme 5**).

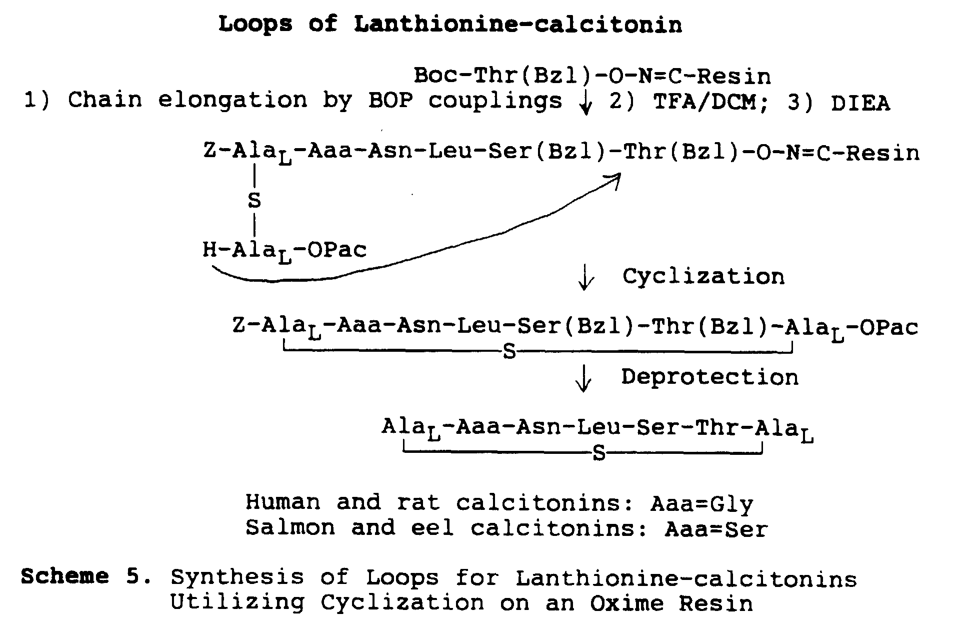

AlaL-Aaa-Asn-Leu-Ser-Thr-AlaL
S
Human and rat calcitonins: Aaa=Gly
Salmon and eel calcitonins: Aaa=Ser

The elongation at the C-terminus to get the final calcitonin-analog can be performed by normal classical fragment condensations or by the strategy shown later in the paragraph of lanthionine-oxytocin and -vasopressin synthesis (**Scheme 6**).

### Lanthionine-oxytocins and Lanthionine-vasopressins

The incorporation of a lanthionine bridge to replace the existing disulfide bridge found in natural oxytocin (OT) and vasopressin (VP) is another example. This can be accomplished by the synthesis of the lanthionine component prior to its incorporation in the peptide sequence (**Scheme 6**).

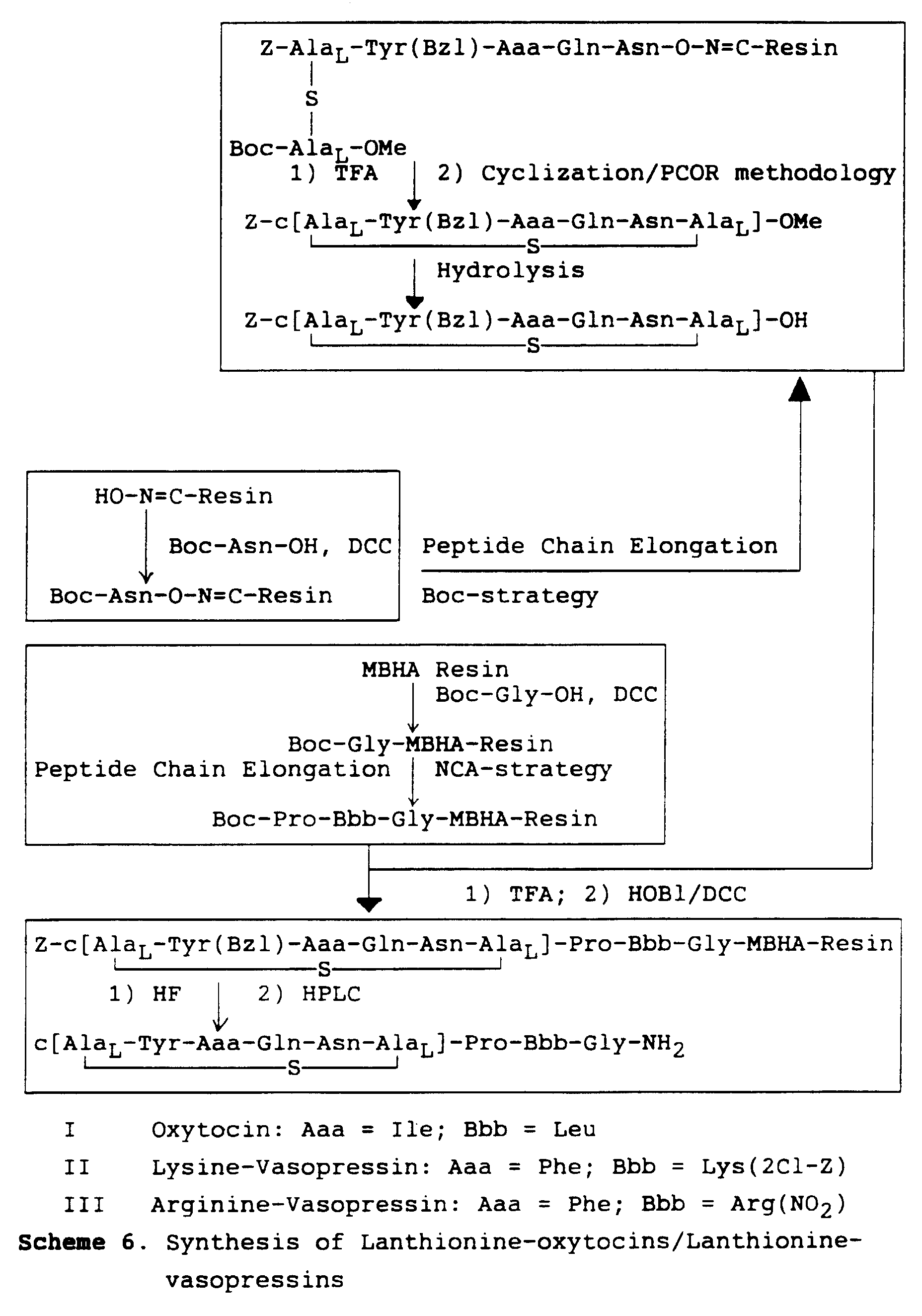

1        2    3     4     5     6     7     8    9
H-AlaL-Tyr-Ile-Gln-Asn-AlaL-Pro-Leu-Gly-NH2 - lanthionine-
      S                                        oxytocin

1        2      3     4     5    6      7     8     9
H-AlaL-Tyr-Phe-Gln-Asn-AlaL-Pro-Lys-Gly-NH2
        S
                                                    lanthionine-
1        2      3     4     5    6      7     8     9      vasopressin
H-AlaL-Tyr-Phe-Gln-Asn-AlaL-Pro-Arg-Gly-NH2
        S

### Example 1:

### a) Preparation of Z-Tyr(Bzl)-Ser-Gly-Phe-Cys(Fm)-MBHA (1)

Methyl benzhydrylamine resin (3 g) was reacted with Boc-Cys(Fm)OH (1.0 g, 2.5 mmol) and DCC (0.52 g, 2.5 mmol) in DCM (30 mL) for 3 hr at room temperature in an SPPS vessel. The remaining amino groups were capped by acetylation. The resulting Boc-Cys(Fm)-MBHA resin (substitution level 0.36 mmol/g, based on picric acid titration) was then deprotected with 30% TFA/DCM (v/v) and neutralized with 1% DIEA/DCM (v/v) solution. The peptide chain was then assembled by consecutive addition of the symmetrical anhydrides (2.5 equiv.) of BocPheOH, BocGlyOH, BocSerOH, and ZTyr(Bzl)OH as well as deprotection steps. The completeness of coupling was monitored by the Kaiser test. Coupling of ZTyr(Bzl)OH was repeated with 1 molar equivalent reagent. Yield 1.06 mmol (84%) peptide based on Gly; amino acid analysis; Cys₍₁₎Gly_{1.00}Phe_{0.86}Ser_{1.42}Tyr_{1.22}. 
b) Preparation of Z-Tyr(Bzl)-c(D-Ala_L-Gly-Phe-Ala_L)-MBHA
S
(3)
 The protected peptidyl MBHA resin (1, 1.06 mmol) in the SPPS vessel was swollen and then suspended in DCM (20 mL). A solution of DSC (387 mg, 1.51 mmol) in acetonitrile (10 mL) was added to the reaction mixture followed by a 5% DIEA/DCM solution (5.22 mL, 1.5 mmol DIEA). The reaction was allowed to proceed for 4 hr, shaking at room temperature in a nitrogen atmosphere. The reaction mixture was drained and the solid phase was washed with DCM (4x). The product (**2**) was treated with a solution of 20% piperidine/DMF solution (20 mL, v/v, 2x50 min.) and shaken in a 5% piperidine/DMF-DCM solution (40 mL, 1/1, v/v) overnight. The solution phase was drained and the resin was washed with DMF (1x), DCM (2x) and EtOH (2x) and dried. Yield 3.7 g.

c) Preparation of H-Tyr-c(D-AlaL-Gly-Phe-AlaL)-NH2 (4)
S

The peptidyl resin (**3**, 1.0 g) was treated with anhydrous HF (20 mL) at 0°C in the presence of anisole (1 mL) for 1 hr in a teflon HF apparatus. After removal of volatile components the remaining material was washed with EtOAc (2x20 mL) and the product was extracted with acetic acid followed by 10% acetic acid/water solution. The combined extracts were freeze-dried (yield 200 mg). This material was purified by preparative RP-HPLC on a Vydac C-18 column (1.0 x 25 cm) eluted with 0.1% TFA in acetonitrile/water. A linear gradient from 15% to 22% acetonitrile over 12 min with a flow rate of 10 mL/min was employed. The appropriate fractions were lyophilized to give a solid product (yield 87 mg). Finally, 30 mg of the product was subjected to gel permeation chromatography (1.5 x 100 cm Sephadex G-15 eluted with 20% acetic acid). The peptide fractions were pooled and lyophilized. Yield 16 mg (24% calculated for compound 1). R_{F}(A) 0.44; R_{F}(B) 0.49. FAB-MS m/e = 557 (M + 1). Amino acid analysis Gly_{1.00}Ala_{L}-S-Ala_{L1.1}Phe_{0.99}Tyr_{0.95}.

### Example 2:

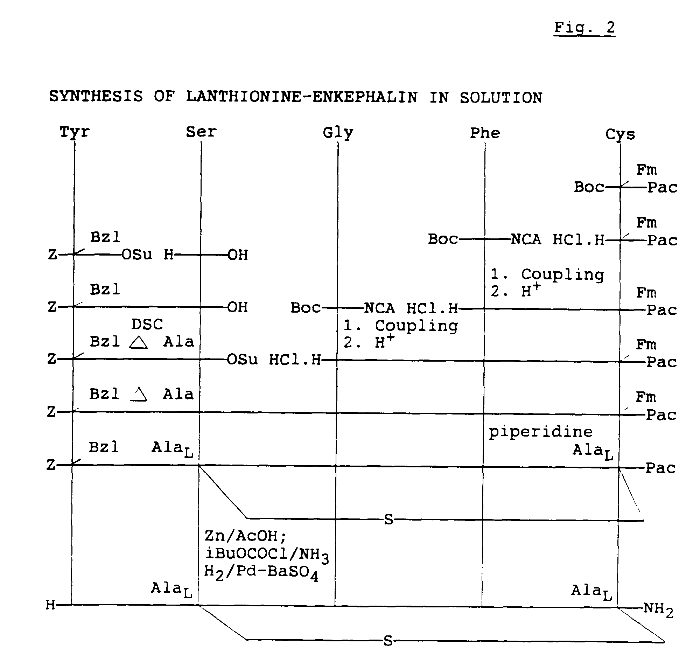

Fig. 2 shows schematically the synthesis of lanthionine-enkephalin in solution. Other general methods for chemical synthesis can be followed using mixed anhydrides, carbodiimides, active esters and other coupling procedures. Preferred solvents are CH₂Cl₂ and DMF. Cleavages are following standard selective reactions. Purification follows well-known extractions, precipitations and chromatographic methods.

### Example 3:

The lanthionine-enkephalin was also synthesized by the preferred Fmoc-NCA-Strategy by using the following steps:

| | | |
|---|---|---|
| (1) | Deprotection (20% piperidine/DMF) | 7 min 30 mL/min |
| (2) | Wash (DMF) | 5 min 30 mL/min |
| (3) | Coupling (see below) | 20 min 30 mL/min |
| (4) | Wash-(DMF) | 5 min 30 mL/min |
| (5) | Repeat steps 1-4 | |

The coupling was performed as follows: #1: 3 eq. 20 min; #2: 1 eq. + DIEA 20 min
(a) Fmoc-Phe-NCA,
(b) Fmoc-Gly-NCA,
(c) Fmoc-Ser-OH/DCC,
(d) Fmoc-Tyr(Bzl)-NCA.

In the case of peptide chain elongation by the Fmoc-strategy the -SH group of cystein was blocked with a Trt group.

### Example 4:

The preparation of lanthionine-bridged cyclic peptide fragments is demonstrated by the following preparations: 
a)  Z-D-Ala_{L}-Phe-D-Ala_{L}-OPac
S
 
Z-D-Ala_{L}-(Boc-D-Ala_{L}-OPac)-Phe-O-oxime
S
 resin (1.0 g, 187 µmol peptide on resin) synthesized by regular solid phase synthetic method was swollen in DCM (10 mL) in a solid-phase peptide synthesis vessel. The Boc group was removed with 25% TFA/DCM, shaking the reaction vessel for 30 min. The peptidyl resin was then drained and washed (10 mL/wash) with DCM (2x), *i*-PrOH (1x), DCM (2x), *i*-PrOH (1x), and DCM (2x). The amino group was neutralized by treating the peptidyl resin with 5% DIEA in DCM (2 x 1 min.) and then washing with DCM (2x). The cyclization reaction was then carried out by shaking the peptidyl resin in DCM/DMF (1:1, v/v, 10 mL) in the presence of 10 equiv. AcOH at RT for 72 h. The cyclic peptide product was collected from the reaction vessel by draining and then washing the resin with DMF (3x). These solutions were combined and evaporated to a reduced volume, and then washed with water, 0.1 N HCl, 5% NaHCO₃, and brine. The solvent was then evaporated and the crude product was purified by silica gel flash chromatography (2 x 20 cm, ethyl acetate-hexanes 1/1). The appropriate fractions were pooled and the solvent was evaporated. The pure solidified product was recrystallized from methanol/ether. Yield 40.5 mg (36.7%); mp 241-244°C (decomp); R_{F}(EtOAc/hexanes = 2/1) 0.42; FAB-MS m/e = 590 (MH⁺); theoretical 590. 
b) Z-L-Ala_{L}-Phe-D-Trp(For)-Lys(2Cl-Z)-Thr(Bzl)-L-Ala_{L}-OPac
S
Z-D-Ala_{L}(Boc-D-Ala_{L}-OPac)-Phe-D-Trp(For)-Lys(2Cl-Z)-
S
Thr(Bzl)-O-oxime
 Thr(Bzl)-O-oxime resin (100 mg, 6.0 µmol peptide on resin) synthesized by regular solid phase synthetic method was swollen in DCM (1.0 mL) in a solid-phase peptide synthesis vessel. The Boc group was removed with 25% TFA/DCM, shaking the reaction vessel for 30 min. The peptidyl resin was then drained and washed (1.0 mL/wash) with DCM (2x), *i*-PrOH (1x), DCM (2x), *i*-PrOH (1x), and DCM (2x). The amino group was neutralized by treating the peptidyl resin with 2.5% DIEA in DCM (2 x 1 min.) and then washing with DCM (2x). The cyclization reaction was then carried out by shaking the peptidyl resin in DCM/DMF (1:2, v/v, 1.0 mL) in the presence of 10 equiv. AcOH at RT for 72 h. The cyclic peptide product was collected from the reaction vessel by draining and then washing the resin with DMF (3x). These solutions were combined and evaporated and the crude product was purified by RP-HPLC on a Vydac C-18 column (1.0x25 cm) using 0.1% TFA in acetonitrile water. A linear gradient from 50 to 80% acetonitrile over 15 min., with a flow rate of 4 mL/min., was employed. The product was eluted at 61% acetonitrile and lyophilized to give a solid product. Yield 0.9 mg (24%); R_{F}(CHCl₃/MeOH/AcOH = 18/1.5/1) 0.54; FAB-MS m/e = 1,293 (MH⁺); theoretical 1,293.

### Example 5:

Comparative examples showing the superior biological activity of compounds with the thioether bond compound compared with the compound having the disulfide bridge:

### Bioassays Using Isolated Organ Preparations

All of these assays represent standard procedures which have been well described in the literature.
(1) The GPI (guinea pig ileum) assay was performed according to a modified version of a procedure first developed by Paton. Male guinea pigs (300-450 g) were killed by a blow on the skull and exsanguinated. A 2-3 cm segment of ileum not less than 10 cm from the ileocecal junction was mounted in a 20 ml organ bath. The bath contained Krebs' solution of the following composition (in millimolar concentrations): NaCl, 150; KCl, 4.3; CaCl₂, 1.25; MgCl₂, 1.0; NaH₂PO₄.H₂O, 1.7; NaHCO₃, 25.0; glucose, 11.0. The temperature was maintained at 37°C and the solution was bubbled with 95% O₂/% CO₂. A GRASS E 2B electrode was used as anode with the 1.5 cm platinum wire entirely enclosed within the lumen. The other end of the preparation was tied over a piece of stiff polyethylene tubing (4 cm, 2.5 mm O.D) which projects out of the bath solution and was tied to the strain gauge. Another GRASS E 2B electrode was placed about 5 mm from the intestine and parallel to it to achieve transmural stimulation. Single pulses of 4 msec during were delivered by a Harvard apparatus stimulator at a frequency of 10 min⁻¹. Voltages in the range from 3 to 6 V were applied in order to obtain maximal response. Isometric contractions of the ileum were recorded via a Harvard isometric force transducer on a Harvard apparatus biograph which has been calibrated to produce a pen displacement of 1 cm per tension change of 1 g. The results were standardized by expressing the reduction in tension obtained at each dose level as a percentage of the mean tension produced by at least ten preceding control stimulations. Semilogarithmic plots of percent inhibition as a function of peptide concentration permit the determination of IC50-values which were taken as the intercept of 50% inhibition.
(2) The MVD (mouse vas deference) assay was performed essentially as described by Henderson. Briefly, adult, male albino mice (Swiss Webster 30-50 g) are killed by cervical dislocation and the vas deferentia are dissected out. After removal of extraneous fat and connective tissue, the vas is stripped of its associated blood vessel and the somen is gently expressed from the lumen. The vas is then mounted under 0.5 g tension in a 5 ml organ bath containing warmed (37°C), oxygenated (95% O₂, 5% CO₂%) , Mg²⁺-free Krebs solutions of the following composition [mM]: NaCl, 118; CaCl₂, 2.54; KCl, 4.75; KH₂PO₄, 1.19; NaHCO₃, 25; glucose, 11; L-tyrosine, 0.2. A modified Harvard apparatus stimulator is used to deliver repetitive field stimulation through platinum wire ring electrodes at the top and bottom of the bath, consisting of twin, rectangular pulses (80 V, 0.15 Hz, 10-ms delay, 1.0-ms duration). Contractions of the muscle are recorded via a Hewlett-Packard Model FTA-1-1 force transducer connected to a Hewlett-Packard 7702B recorder. Determination of the reduction in the twitch height at various doses permits the construction of log dose-response curves and the determination of IC50-values.

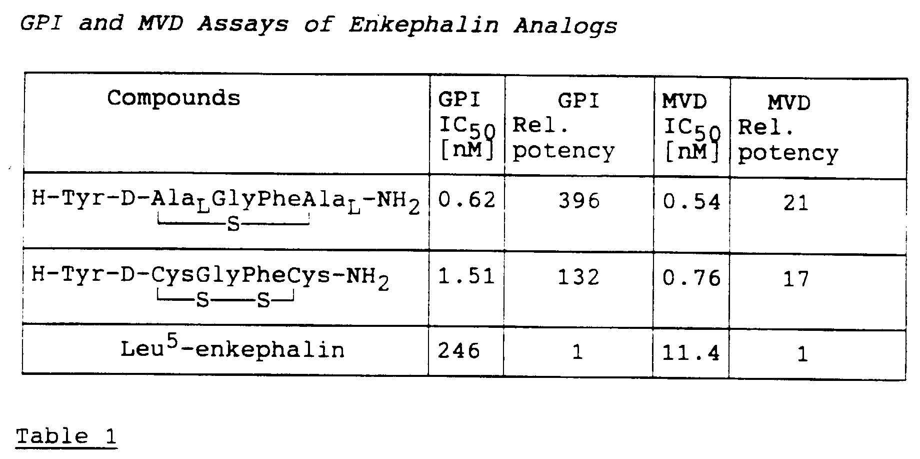

Table 1 shows that the compound according to the invention which has a thioether bond is in both methods, particularly in the most relevant GPI test more potent than the corresponding -S-S- compound.

### Example 6:

Using protocols described by Schiller et al., Biochem. Biophys. Res. Commun. 1983, 115, p. 864-870, we compared the half-lives of three compounds: Leu⁵-enkephalin, disulfide-enkephalin, and lanthionine-enkephalin. As indicated in **Table 2**, the lanthionine-enkephalin is much more stable than the other two compounds.

**Table 2.**

| Enzymatic Degradation of Enkephalin Analogs | |
|---|---|
| Analog | t_{1/2} (min) |
| Lanthionine-enkephalin | 1223 |
| Disulfide-enkephalin | 332 |
| Leu⁵-enkephalin | 30 |

### Example 7:

The lanthionine opioid is highly active in the *in vitro* and *in vivo* tests (**Table 3**). *In vivo* bioactivity was determined using the rat hot plate test after intrathecal dosages. 
S
Tyr-c[D-AlaL-Gly-Phe-AlaL]-NH2
 shows 37 times higher bioactivity than morphine and twice the activity of DCLCE (**Table 3**). In the same tests, [Leu⁵]-enkephalin shows only 40-50% of full agonistic activity after 100 µg dosage in the *in vitro* assays using GPI and MVD preparations. The lanthionine opioid exhibits 400 times greater bioactivity at the GPI (µ-receptor) and 20 times greater bioactivity at the MVD (δ-receptor) than [Leu⁵]-enkephalin. These values are higher than those of its disulfide bridged counterpart, DCDCE. The lanthionine analog does not show a preference for the µ- or the δ -receptor. The IC₅₀ ratio (MVD/GPI) is 0.9.

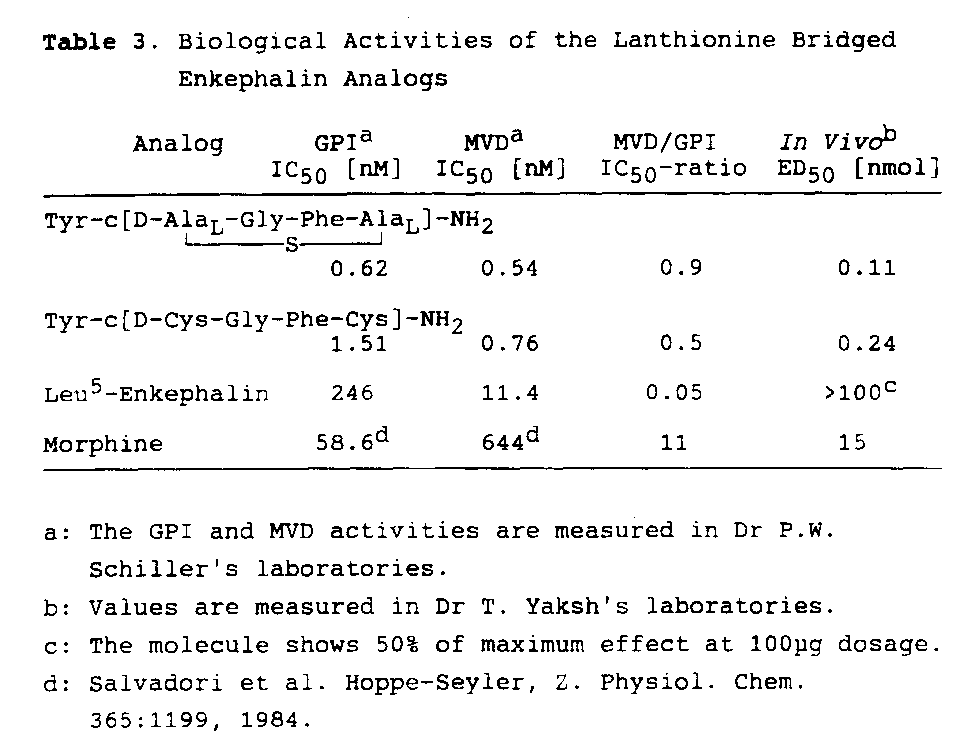

Although the lanthionine-enkephalins according to the invention possess superactivity, they do not seem to have high receptor selectivity. To improve their selectivity, it is possible to introduce one or more alkyl (methyl) group(s) in β-position(s) of the lanthionine segment.
In this case at least one of R₄, R₅, R₇, R₈ may be alkyl (methyl).

## Claims

1. Lanthionine-bridged peptides with the general formula 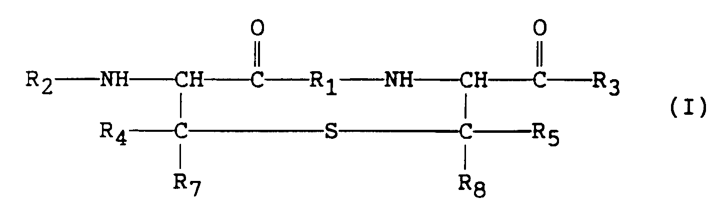 wherein R₁ is a short sequence of 2 to 10 amino acids selected from the group of the naturally occurring amino acids and the D-enantiomers thereof and
R₂ and R₃ respectively represent naturally occurring amino acids as L- or D-enantiomers or a short sequence of up to 25, preferably 3 amino acids, wherein the N-terminal -NH₂ group of the R₂ residue may be replaced by -OH, -H or -NHCOR₆ wherein R₆ is an alkyl- or aralkylresidue or the C-terminal -COOH of the R₃ amino acid residue may be replaced by -CONH₂ or -CH₂OH or R₂ may represent -H, acyl or aracyl having 1-18 carbon atoms and R₃ may be OH or NH₂ and 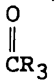 may be replaced by CH₂OH with the proviso that R₁ is not Phe-Trp-Lys-Thr, when R₂ is Phe and R₃ is Thr(ol), whereby R₁, R₂ or R₃ can also comprise peptidomimetics such as retroinverso-, carba-, aza-, thiopeptides or peptide rings and wherein R₄, R₅, R₇ and R₈ are hydrogen or an alkyl having 1 to 10 carbon atoms.

2. Peptides according to claim 1, wherein R₁ is a short sequence of 2 to 7, especially 2 to 4, amino acids.

3. Peptides according to claim 1 or 2, wherein R₂ and R₃ represent one amino acid selected independently from each other from the group consisting of D-Phe, D-β-Nal, Tyr, TrpNH₂, ThrNH₂, Thr(ol) or represent the amino acid sequence of Pro-Arg-Gly or Pro-Leu-Gly or wherein R₂ is -H, acyl or aracyl and R₃ is -OH or -NH₂ and the group 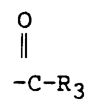 can be replaced by CH₂OH.

4. Peptides according to claim 3, wherein R₁ represents an amino acid sequence selected from the group consisting of Gly-Phe, Phe-D-Trp-Lys-Val, Phe-D-Trp-Lys-Thr, Tyr-D-Trp-Lys-Val, Tyr-Phe-Gln-Asn, Tyr-Ile-Gln-Asn, Gly-Asn-Leu-Ser-Thr, Ser-Asn-Leu-Ser-Thr or Glu-Lys-Asp-Met-Leu-Ser-Ser.

5. Peptides according to claim 4 having the general formula 
H-Tyr-D/L-Ala_{L}-Gly-Phe-D/L-Ala_{L}-R₃
S
 wherein R₃ is OH or NH₂.

6. Peptides according to claim 4 having the general formula 
H-Xxx-Ala_{L}-Phe-D-Trp-Lys-Yyy-Ala_{L}-Zzz
S
 wherein Xxx represents D-Phe, D-β-Nal; Yyy is Thr or Val, and Zzz is TrpNH₂, ThrNH₂ or Thr(ol) with the proviso that Xxx is not D-Phe if Zzz is Thr(ol).

7. Peptides according to claim 4 having the general formula 
H-DPhe-Ala_{L}-Tyr-DTrp-Lys-Val-Ala_{L}-Trp-NH₂
S
 or

H-DßNal-AlaL-Tyr-DTrp-Lys-Val-AlaL-Thr-NH2
S

8. Peptides according to claim 4 having the general formula 
H-Ala_{L}-Tyr-Aaa-Gln-Asn-Ala_{L}-Pro-Bbb-Gly-NH₂
S
 wherein Aaa is Phe or Ile and Bbb is Arg or Leu.

9. Peptides according to claim 4 having the general formula 
R2-AlaL-Ggg-Asn-Leu-Ser-Thr-AlaL-R3
S
 wherein Ggg is Gly or Ser, R₂ is -H, acyl or aracyl each having 1-18 carbon atoms and R₃ represent the fragment 8-32 of human, salmon or eel-calcitonin.

10. Peptides according to claim 1 having the amino acid sequence of endothelin or related peptides wherein one or two of the two disulfide bridges are replaced by one or two thioether bonds and where the rings are sequentially overlapping.

11. Pharmaceutical composition characterized in that it contains an effective amount of at least one peptide of the general formula 
O                                    O
‖                                    ‖
R2—NH—CH—C—R1—NH—CH—C—R3          (I)
|                    |
R4—C————S————C—R5
|                    |
R7                   R8
 wherein R₁ is a short sequence of 2 to 10 amino acids selected from the group of the naturally occurring amino acids and the D-enantiomers thereof and
R₂ and R₃ respectively represent naturally occurring amino acids as L- or D-enantiomers or a short sequence of up to 25, preferably three amino acids, wherein the N-terminal -NH₂ group of the R₂ residue may be replaced by -OH, -H or -NHCOR₆ wherein R₆ is an alkyl- or aralkylresidue or the C-terminal -COOH may be replaced by -CONH₂ or -CH₂OH or wherein R₂ represents -H, acyl or aracyl and R₃ may be -OH or -NH₂ and 
O
||
-CR3
 may be replaced by CH₂OH whereby R₁, R₂ or R₃ can also comprise peptidomimetics such as retro-inverso-, carba-, aza-, thiopeptides or peptide rings and R₄, R₅, R₇ and R₈ represent hydrogen or an alkyl having 1 to 10 carbon atoms.

12. Pharmaceutical composition characterized in that it contains an effective amount of at least one peptide according to any one of claims 2 to 10.

13. Process for the preparation of a peptide as claimed in any one of claims 1 to 10 using an appropriate combination of solid-phase peptide synthesis and/or classical synthesis, wherein at least one of the peptide fragments contains a moiety which is cyclized either attached to the resin used or after cleavage from the resin to the desired lanthionine-bridged cyclic peptide fragment which can optionally be elongated at the N- and/or C-terminal to form the final peptide by fragment condensation or step by step synthesis.

14. Process for the preparation of a peptide as claimed in claim 13 characterized in that
- the peptide fragments containing the moiety to be cyclized is assembled on an appropriate resin using *tert*-butoxycarbonyl-chemistry with any peptide coupling method,
- serine is incorporated at the desired place, which is then converted to dehydroalanine using disuccinimido carbonate,
- the S-protecting group attached to the cysteine coupled at the desired place is selectively removed,
- the Michael addition of the SH group to the double bound is promoted by a slightly basic milieu and
- the peptide and the other protecting groups are cleaved from the resin by treatment with HF.

15. Process according to claim 14 characterized by assembling the peptide chain at any appropriate resin using the Fmoc-strategy with any usable coupling agent, intermediately using cleavage of the Fmoc-protecting-group by the piperidine-method, wherein the cleavage of the acid labile S-protecting-group is carried out by any appropriate acid or reagent.

16. Process of preparation of a peptide as claimed in any one of the claims 13 to 15, using the classical synthesis in solution as fragment-condensation or as a step by step synthesis, wherein any known coupling method of appropriately protected amino acids and any cleavage-method of fragments and the final peptide is used.

## Patentansprüche

1. Lanthionin-verbrückte Peptide mit der allgemeinen Formel 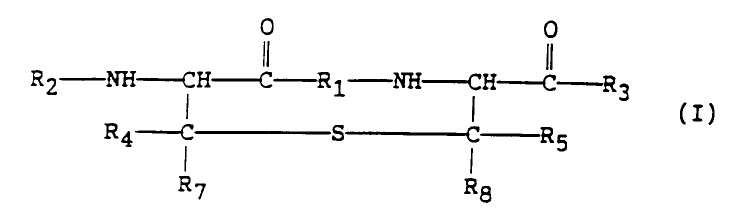 worin R₁ eine kurze Sequenz von 2 bis 10 Aminosäuren ist, ausgewählt aus der Gruppe der natürlich vorkommenden Aminosäuren und der D-Enantiomere davon, und
R₂ und R₃ jeweils natürlich vorkommende Aminosäuren als L- oder D-Enantiomere oder eine kurze Sequenz von bis zu 25, bevorzugt 3 Aminosäuren, darstellen, worin die N-terminale -NH₂-Gruppe des R₂-Restes durch -OH, -H oder -NHCOR₆ ersetzt sein kann, worin R₆ ein Alkyl- oder Aralkylrest ist, oder das C-terminale -COOH des R₃-Aminosäurerests durch -CONH₂ oder -CH₂OH ersetzt sein kann, oder R₂ -H, Acyl oder Aracyl mit 1 - 18 Kohlenstoffatomen darstellen kann, und R₃ OH oder NH₂ sein kann, und 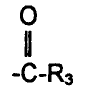 durch CH₂OH ersetzt sein kann, mit der Vorgabe, daß R₁ nicht Phe-Trp-Lys-Thr ist, wenn R₂ Phe ist und R₃ Thr(ol) ist, wobei R₁, R₂ oder R₃ auch peptidomimetische Gruppen umfassen können, wie Retro-Inverso-, Carba-, Aza-, Thiopeptide oder Peptidringe, und worin R₄, R₅, R₇ und R₈ Wasserstoff oder ein Alkyl mit 1 bis 10 Kohlenstoffatomen sind.

2. Peptide nach Anspruch 1, worin R₁ eine kurze Sequenz von 2 bis 7, insbesondere 2 bis 4 Aminosäuren ist.

3. Peptide nach Anspruch 1 oder 2, worin R₂ und R₃ eine Aminosäure darstellen, unabhängig voneinander ausgewählt aus der Gruppe, bestehend aus D-Phe, D-β-Nal, Tyr, TrpNH₂, ThrNH₂, Thr(ol) oder die Aminosäuresequenz Pro-Arg-Gly oder Pro-Leu-Gly darstellen oder worin R₂ -H, Acyl oder Aracyl ist und R₃ -OH oder -NH₂ ist und die Gruppe 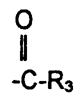 durch CH₂OH ersetzt sein kann.

4. Peptide nach Anspruch 3, worin R₁ eine Aminosäuresequenz darstellt, ausgewählt aus der Gruppe, bestehend aus Gly-Phe, Phe-D-Trp-Lys-Val, Phe-D-Trp-Lys-Thr, Tyr-D-Trp-Lys-Val, Tyr-Phe-Gln-Asn, Tyr-Ile-Gln-Asn, Gly-Asn-Leu-Ser-Thr, Ser-Asn-Leu-Ser-Thr oder Glu-Lys-Asp-Met-Leu-Ser-Ser.

5. Peptide nach Anspruch 4 mit der allgemeinen Formel 
H-Tyr-D/L-Ala_{L}-Gly-Phe-D/L-Ala_{L}-R₃
S
 worin R₃ OH oder NH₂ ist.

6. Peptide nach Anspruch 4 mit der allgemeinen Formel 
H-Xxx-Ala_{L}-Phe-D-Trp-Lys-Yyy-Ala_{L}-Zzz
S
 worin Xxx D-Phe, D-β-Nal darstellt; Yyy Thr oder Val ist und Zzz TrpNH₂, ThrNH₂ oder Thr(ol) ist, mit der Vorgabe, daß Xxx nicht D-Phe ist, wenn Zzz Thr(ol) ist.

7. Peptide nach Anspruch 4 mit der allgemeinen Formel 
H-DPhe-AlaL-Tyr-DTrp-Lys-Val-AlaL-Trp-NH2
S
 oder

H-DβNal-AlaL-Tyr-DTrp-Lys-Val-AlaL-Thr-NH2
S

8. Peptide nach Anspruch 4 mit der allgemeinen Formel 
H-Ala_L-Tyr-Aaa-Gln-Asn-Ala_L-Pro-Bbb-Gly-NH_2
S
 worin Aaa Phe oder Ile ist und Bbb Arg oder Leu ist.

9. Peptide nach Anspruch 4 mit der allgemeinen Formel 
R2-AlaL-Ggg-Asn-Leu-Ser-Thr-AlaL-R3
S
 worin Ggg Gly oder Ser ist, R₂ -H, Acyl oder Aracyl mit jeweils 1 - 18 Kohlenstoffatomen ist und R₃ das Fragment 8 - 32 von human, Lachs- oder Aal-Calcitonin darstellt.

10. Peptide nach Anspruch 1 mit der Aminosäuresequenz von Endothelin oder verwandten Peptiden, worin eine oder zwei der zwei Disulfidbrücken durch eine oder zwei Thioetherbindungen ersetzt sind und worin die Ringe sequentiell überlappend sind.

11. Pharmazeutische Zusammensetzung, dadurch gekennzeichnet, daß sie eine wirksame Menge mindestens eines Peptids der allgemeinen Formel 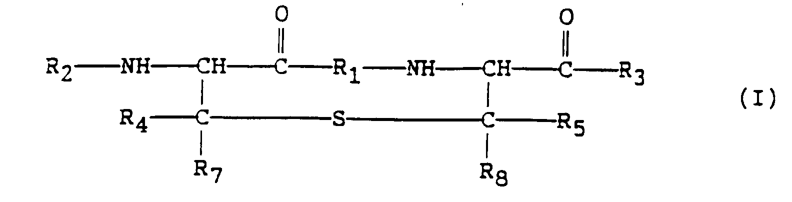 enthält, worin R₁ eine kurze Sequenz von 2 bis 10 Aminosäuren ist, ausgewählt aus der Gruppe der natürlich vorkommenden Aminosäuren und der D-Enantiomere davon und
R₂ und R₃ jeweils natürlich vorkommende Aminosäuren als L- oder D-Enantiomere oder eine kurze Sequenz von bis zu 25, bevorzugt drei Aminosäuren, darstellen, worin die N-terminale -NH₂-Gruppe des R₂-Restes durch -OH, -H oder -NHCOR₆ ersetzt sein kann, worin R₆ ein Alkyl- oder Aralkylrest ist, oder das C-terminale -COOH durch -CONH₂ oder -CH₂OH ersetzt sein kann, oder worin R₂ -H, Acyl oder Aracyl darstellt und R₃ -OH oder -NH₂ sein kann, und 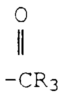 durch CH₂OH ersetzt sein kann, wobei R₁, R₂ oder R₃ auch peptidomimetische Gruppen wie Retro-Inverso-, Carba-, Aza-, Thiopeptide oder Peptidringe umfassen können und R₄, R₅, R₇ und R₈ Wasserstoff oder ein Alkyl mit 1 bis 10 Kohlenstoffatomen darstellen.

12. Pharmazeutische Zusammensetzung, dadurch gekennzeichnet, daß sie eine wirksame Menge mindestens eines Peptids nach einem der Ansprüche 2 bis 10 enthält.

13. Verfahren zur Herstellung eines Peptids wie in einem der Ansprüche 1 bis 10 beansprucht unter Verwendung einer geeigneten Kombination aus Festphasenpeptidsynthese und/oder klassischer Synthese, worin mindestens eines der Peptidfragmente eine Gruppe enthält, die entweder an das verwendete Harz gebunden oder nach Abspaltung von dem Harz zu dem gewünschten Lanthionin-verbrückten cyclischen Peptidfragment cyclisiert wird, das gegebenenfalls an dem N- und/oder C-Terminus zur Bildung des Endpeptids durch Fragmentkondensation oder Schritt-bei-Schritt-Synthese verlängert werden kann.

14. Verfahren zur Herstellung eines Peptids wie in Anspruch 13 beansprucht, dadurch gekennzeichnet, daß
- das Peptidfragment, das die zu cyclisierende Gruppe enthält, an einem geeigneten Harz unter Verwendung der *tert*-Butoxycarbonylchemie mit irgendeiner Peptidverbindungsmethode zusammengefügt wird,
- Serin am gewünschten Platz eingebaut wird, das dann unter Verwendung von Disuccinimidocarbonat zu Dehydroalanin umgewandelt wird,
- die S-Schutzgruppe, die an das Cystein gebunden ist, das an dem gewünschten Platz gebunden ist, selektiv entfernt wird,
- die Michael-Addition der SH-Gruppe an die Doppelbindung durch ein leicht basisches Milieu begünstigt wird und
- das Peptid und die anderen Schutzgruppen durch Behandlung mit HF von dem Harz abgespalten werden.

15. Verfahren nach Anspruch 14, gekennzeichnet durch das Zusammenfügen der Peptidkette an jedem geeigneten Harz unter Verwendung der Fmoc-Strategie mit irgendeinem verwendbaren Verbindungsmittel, unter zwischenzeitlichem Einsatz einer Abspaltung der Fmoc-Schutzgruppe durch die Piperidinmethode, worin die Abspaltung der säure labilen S-Schutzgruppe durch irgendeine geeignete Säure oder Reagenz ausgeführt wird.

16. Verfahren zur Herstellung eines Peptids wie in einem der Ansprüche 13 bis 15 beansprucht unter Verwendung der klassischen Synthese in Lösung als Fragmentkondensation oder als eine Schritt-bei-Schritt-Synthese, worin irgendeine bekannte Verbindungsmethode von geeignet geschützten Aminosäuren und irgendeine Abspaltungsmethode von Fragmenten und dem Endpeptid verwendet werden.

## Revendications

1. Peptides pontés par de la lanthionine, qui répondent à la formule générale suivante : 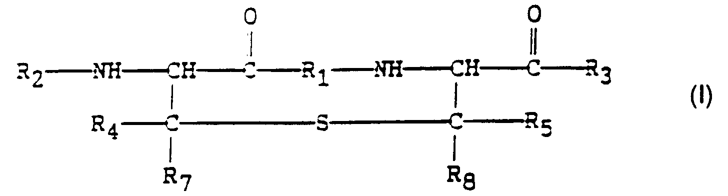 dans laquelle
R₁ représente une courte séquence de 2 à 10 aminoacides, choisis dans le groupe formé par les aminoacides naturels et leurs énantiomères D, et
R₂ et R₃ représentent respectivement des aminoacides naturels, comme les énantiomères L ou D, ou une courte séquence allant jusqu'à 25, de préférence, 3, aminoacides, où le groupe -NH2 N-terminal du reste R₂ peut être remplacé par un groupe -OH, -H ou -NHCOR₆, où R₆ est un reste alkyle ou aralkyle, ou bien le groupe -COOH C-terminal du reste aminoacide R₃ peut être remplacé par un groupe -CONH₂ ou -CH₂OH, ou bien R₂ peut représenter -H, un radical acyle ou aracyle, possédant de 1à 18 atomes de carbone et R₃ peut être un groupe -OH ou -NH₂ et 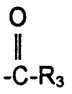 peut être remplacé par CH₂OH avec la condition que R₁ ne soit pas Phe-Trp-Lys-Thr, lorsque R₂ est Phe et R₃ est Thr(ol), où R₁, R₂ ou R₃ peuvent également comprendre des peptidomimétiques, comme des rétro-inverso-, carba-, aza-, thiopeptides ou des noyaux peptide et où R₄, R₅, R₇ et R₈ représentent des atomes d'hydrogène ou des radicaux alkyle possédant de 1 à 10 atomes de carbone.

2. Peptides suivant la revendication 1, caractérisés en ce que R₁ est une courte séquence de 2 à 7 aminoacides, plus particulièrement, de 2 à 4 aminoacides.

3. Peptides suivant la revendication 1 ou 2, caractérisés en ce que R₂ et R₃ représentent un aminoacide chacun indépendamment choisi dans le groupe formé par D-Phe, D-β-Nal, Tyr, TrpNH₂, ThrNH₂, Thr(ol), ou représentent la séquence d'aminoacides de Pro-Arg-Gly ou Pro-Leu-Gly, ou bien R₂ est -H, acyle ou aracyle et R₃ est -OH ou -NH₂ et le groupe 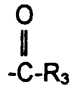 peut être remplacé par CH₂OH.

4. Peptides suivant la revendication 3, caractérisés en ce que R₁ représente une séquence d'aminoacides choisis dans le groupe formé par Gly-Phe, Phe-D-Trp-Lys-Val, Phe-D-Trp-Lys-Thr, Tyr-D-Trp-Lys-Val, Tyr-Phe-Gln-Asn, Tyr-Ile-Gln-Asn, Gly-Asn-Leu-Ser-Thr, Ser-Asn-Leu-Ser-Thr ou Glu-Lys-Asp-Met-Leu-Ser-Ser.

5. Peptides suivant la revendication 4, répondant à la formule générale : 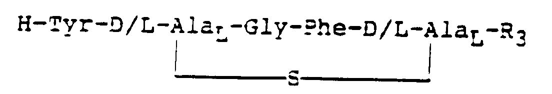
H-Tyr-D/L-AlaL-Gly-Phe-D/L-AlaL-R3
S
 dans laquelle R₃ est OH ou NH₂.

6. Peptides suivant la revendication 4, répondant à la formule générale : 
H-Xxx-Ala_{L}-Phe-D-Trp-Lys-Yyy-Ala_{L}-Zzz
S
 dans laquelle Xxx représente D-Phe, D-β-Nal; Yyy est Thr ou Val, et Zzz est TrpNH₂, ThrNH₂ ou Thr(ol), avec la condition que Xxx ne soit pas D-Phe si Zzz est Thr(ol).

7. Peptides suivant la revendication 4, répondant à la formule générale : 
H-DPhe-AlaL-Tyr-DTrp-Lys-Val-AlaL-Trp-NH2
S
 ou

H-DβNal-AlaL-Tyr-DTrp-Lys-Val-AlaL-Thr-NH2
S

8. Peptides suivant la revendication 4, répondant à la formule générale : 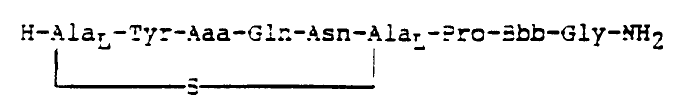
H-AlaL-Tyr-Aaa-Gln-Asn-AlaL-Pro-Bbb-Gly-NH2
S
 dans laquelle Aaa est Phe ou Ile et Bbb est Arg ou Leu.

9. Peptides suivant la revendication 4, répondant à la formule générale : 
R2-AlaL-Ggg-Asn-Leu-Ser-Thr-AlaL-R3
S
 dans laquelle Ggg est Gly ou Ser, R₂ est -H, acyle ou aracyle, chacun comportant de 1 à 18 atomes de carbone et R₃ représente le fragment 8-32 de la calcitonine d'être humain, de saumon ou d'anguille.

10. Peptides suivant la revendication 1, possédant la séquence d'aminoacides de l'endothéline ou des peptides apparentés, où l'un des ou les deux ponts disulfure sont remplacés par une ou deux liaisons thioéther et où les noyaux ou cycles sont séquentiellement chevauchants.

11. Composition pharmaceutique, caractérisée en ce qu'elle contient une quantité efficace d'au moins un peptide de la formule générale: 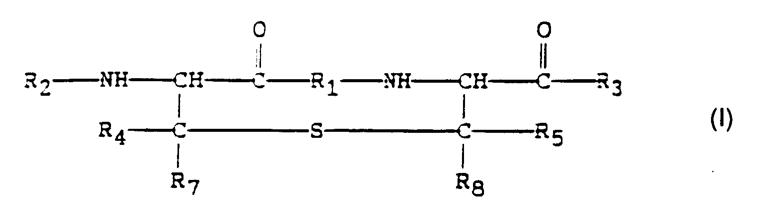 dans laquelle
R₁ représente une courte séquence de 2 à 10 aminoacides, choisis dans le groupe formé par les aminoacides naturels et leurs énantiomères D, et
R₂ et R₃ représentent respectivement des aminoacides naturels, comme les énantiomères L ou D, ou une courte séquence allant jusqu'à 25, de préférence, 3, aminoacides, où le groupe -NH2 N-terminal du reste R₂ peut être remplacé par un groupe -OH, -H ou -NHCOR₆, où R₆ est un reste alkyle ou aralkyle, ou bien le groupe -COOH C-terminal peut être remplacé par un groupe CONH₂ ou -CH₂OH, ou bien R₂ peut représenter -H, un radical acyle ou aracyle et R₃ peut être un groupe -OH ou -NH₂ et 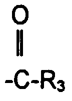 peut être remplacé par CH₂OH où R₁, R₂ ou R₃ peuvent également comprendre des peptidomimétiques, comme des rétro-inverso-, carba-, aza-, thiopeptides ou des noyaux peptide et où R₄, R₅, R₇ et R₈ représentent des atomes d'hydrogène ou des radicaux alkyle possédant de 1 à 10 atomes de carbone.

12. Composition pharmaceutique, caractérisée en ce qu'elle contient une quantité efficace d'au moins un peptide suivant l'une quelconque des revendications 2 à 10.

13. Procédé de préparation d'un peptide suivant l'une quelconque des revendications 1 à 10, en utilisant une combinaison appropriée de synthèse de peptides en phase solide et/ou de synthèse classique, où au moins l'un des fragments peptidiques contient un groupement qui est cyclisé soit attaché à la résine utilisée ou après scission d'avec la résine, au fragment peptidique cyclique ponté à la lanthionine, qui peut éventuellement être allongé au terminal N et/ou C pour former le peptide final par condensation de fragment ou par synthèse étape par étape.

14. Procédé de préparation d'un peptide suivant la revendication 13, caractérisé en ce que
- les fragments peptidiques contenant le groupement à cycliser sont rassembler sur une résine appropriée en utilisant une chimie à tert-butoxycarbonyle avec n'importe quelle méthode de couplage de peptides,
- que la sérine est incorporée à la place souhaitée, que l'on transforme ensuite en déshydroalanine en utilisant du disuccinimidocarbonate,
- le groupe protégeant S attaché à la cystéine couplée à la place souhaitée est sélectivement enlevé,
- l'addition de Michael du groupe SH à la double liaison est favorisée par un milieu légèrement basique, et
- le peptide et les autres groupes protecteurs sont scindés de la résine par traitement par de l'HF.

15. Procédé suivant la revendication 14, caractérisé par l'assemblage de la chaîne peptidique à n'importe quelle résine appropriée en utilisant la stratégie Fmoc avec n'importe quel agent de couplage utilisable, en utilisant de façon intermédiaire la scission du groupe protégeant Fmoc par la méthode à la pipéridine, où la scission du groupe protégeant S labile à l'acide s'entreprend par n'importe quel acide ou réactif convenable.

16. Procédé de préparation d'un peptide suivant l'une quelconque des revendications 13 à 15, en utilisant la synthèse classique en solution, comme par condensation de fragment ou sous forme de synthèse étape par étape, caractérisé en ce que l'on utilise n'importe quelle méthode de couplage connue d'aminoacides protégés de façon appropriée et n'importe quelle méthode de scission de fragment et le peptide final.
